Europäisches Patentamt

European Patent Office

Office européen des brevets

(19)

(11) Veröffentlichungsnummer : **0 201 805**
**B1**

(12) **EUROPÄISCHE PATENTSCHRIFT**

(45) Veröffentlichungstag der Patentschrift :
30.08.89

(21) Anmeldenummer : 86105938.4

(22) Anmeldetag : 30.04.86

(51) Int. Cl.⁴ : **C 12 N 9/96**, C 12 N 9/78,
C 12 N 9/06, C 12 N 11/10,
C 12 Q 1/26, C 12 Q 1/34

(54) Stabilisiertes Sarcosinoxidase-Präparat.

(30) Priorität : 30.04.85 DE 3515586

(43) Veröffentlichungstag der Anmeldung :
20.11.86 Patentblatt 86/47

(45) Bekanntmachung des Hinweises auf die Patenterteilung : 30.08.89 Patentblatt 89/35

(84) Benannte Vertragsstaaten :
AT BE CH DE FR GB IT LI LU NL SE

(56) Entgegenhaltungen :
EP-A- 0 066 552
EP-A- 0 069 379
EP-A- 0 135 070
WO-A-79/006 09
CLINICAL CHEMISTRY, Band 29, Nr. 1, Januar 1983,
Seiten 51-55, Washington, US; T. TSUCHIDA et al.:
"Multi-enzyme membrane electrodes for determination of creatinine and creatine in serum"
CHEMICAL ABSTRACTS, Band 93, Nr. 7, August 1980,
Seite 735, Nr. 68683a, Columbus, Ohio, US; & JP - A -
80 34 001 (NODA INSTITUTE FOR SCIENTIFIC
RESEARCH) 10.03.1980
CHEMICAL ABSTRACTS, Band 97, Nr. 5, August 1982,
Seite 249, Nr. 35365c, Columbus, Ohio, US; & JP - A -
82 36 985 (TOYOBO CO., LTD.) 27.02.1982

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Tischer, Wilhelm, Dr.
Finkenweg 5
D-8123 Peissenberg (DE)
Erfinder : Gloger, Manfred, Dr. rer. nat.
Karwendelstrasse 6
D-8120 Weilheim (DE)
Erfinder : Heinle, Josef
Bayrischzeller Strasse 29
D-8000 München 90 (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

## Beschreibung

Die Erfindung betrifft ein stabilisiertes Sarcosinoxidase-Präparat und dessen Verwendung.

Sarcosinoxidase E.C. 1.5.3.1 (Sarc-OD) katalysiert die Umsetzung von Sarcosin mit $O_2$ und $H_2O$ unter Bildung von Formaldehyd und $H_2O_2$. Es kann aufgrund dieser Reaktion, bei der das $H_2O_2$ leicht aus dem Gleichgewicht entfernt werden kann, für die Bestimmung von Sarcosin verwendet werden. Sarcosin seinerseits entsteht bei der Hydrolyse von Creatin unter der Einwirkung des Enzyms Creatinamidinohydrolase E.C. 3.5.3.3, wobei unter Aufnahme von Wasser Sarcosin und Harnstoff gebildet wird. Da schließlich Creatin aus Creatinin enzymatisch gebildet werden kann, ermöglicht die Sarcosinbestimmung in der oben beschriebenen Weise auch eine Bestimmung von Creatin und von Creatinin. Insbesondere die Bestimmung von Creatinin im Blutserum hat aber erhebliche klinische Bedeutung.

Um die Sarcosinoxidase jedoch in einem Reagenz zur Bestimmung von Sarcosin, Creatin oder Creatinin verwenden zu können, ist es in der Praxis entscheidend, daß das Enzym in getrockneter (lyophilisierter), besonders aber in rekonstituierter gelöster Form eine ausreichende Beständigkeit aufweist. Das Fehlen dieser Eigenschaft hat es bisher verhindert, die Sarcosinoxidase in der erläuterten Weise für die Sarcosinbestimmung in der Praxis einzusetzen. So zeigt die Sarcosinoxidase in gepufferter wäßriger Lösung schon nach einer Stunde eine deutliche Trübung, die bei optischen Meßmethoden besonders berücksichtigt werden muß. Die für ein kommerziell einsetzbares Reagenz erforderliche Mindesthaltbarkeit von einigen Tagen ist nicht gegeben.

Es war bereits aus Chem. Abs. 93 (1980) 735, Abstract Nr. 68 683 a bekannt, Sarcosinoxidase zu stabilisieren, indem man einer wäßrigen Lösung der Sarcosinoxidase einen Zucker, eine Aminosäure oder ein Komplexierungsmittel zugibt und anschließend die Lösung lyophilisiert. Das Lyophilisat ist dann stabil. Weiterhin ist aus Chem. Abs. 97 (1982) 249, Abstract Nr. 35 365 c bekannt, Sarcosinoxidase durch Zugabe von Gluconsäure oder deren Salzen und anschließende Lyophilisierung zu stabilisieren. Mit diesen Verfahren läßt sich jedoch noch keine ausreichende Stabilisierung eines Sarcosinoxidase-Präparates erzielen.

Der Erfindung liegt daher die Aufgabe zugrunde, ein stabilisiertes Sarcosinoxidase-Präparat zu schaffen, welches die genannten Nachteile nicht aufweist und sich für eine kommerzielle Sarcosinbestimmung verwenden läßt.

Gelöst wird diese Aufgabe durch ein stabilisiertes Sarcosinoxidasepräparat, welches gekennzeichnet ist durch einen Gehalt an kovalent an wasserlösliches Polysaccharid gebundene Creatinamidinohydrolase.

Überraschenderweise verhindert die Polysaccharid gebundene Creatinamidinohydrolase die Trübungsbildung durch die Sarcosinoxidase.

Worauf diese Wirkung beruht, ist nicht bekannt. Es erscheint möglich, daß sich die beiden Enzyme komplexartig kombinieren, da das eine Enzym Sarcosin als Produkt liefert, welches vom anderen Enzym direkt als Substrat aufgenommen wird, wobei der Kohlenhydratanteil des einen Enzyms dann das andere Enzym gegen instabilisierende Einflüsse abschirmen könnte.

Werden nämlich die drei Komponenten dieses Präparates ohne kovalente Bindung zwischen dem Polysaccharid und der Creatinamidinohydrolase zusammengegeben, so läßt sich die Trübungsbildung nicht verhindern.

Als geeignete Polysaccharide können lösliche Stärke, Glycogen, Dextran, Inulin, Pectin, Mannan und Galactan genannt werden. Bevorzugt wird Dextran.

Ein besonderer Vorzug des erfindungsgemäßen stabilisierten Enzympräparates besteht darin, daß es auch in Gegenwart von oberflächenaktiven Mitteln, welche die nicht stabilisierte Sarcosinoxidase in der Regel rasch eintrüben, verbesserte Stabilität aufweist. Da oberflächenaktive Mittel in der Regel benötigt werden, wenn Stoffwechselprodukte wie Sarcosin in Körperflüssigkeiten, insbesondere in Serum, bestimmt werden sollen, um eine Trübungsbildung durch andere Serumbestandteile zu verhindern, bedeutet dies einen wesentlichen Vorteil des erfindungsgemäßen Präparates. Das Präparat enthält daher vorzugsweise auch wenigstens ein oberflächenaktives Mittel. Als oberflächenaktive Mittel kommen hierbei in erster Linie die als Bestandteile von sogenannten « Aufhellungssystemen » für Serum bekannten Substanzen in betracht. Typische Beispiele hierfür sind nichtionogene oberflächenaktive Substanzen und anionische oberflächenaktive Substanzen, insbesondere aus der Cholsäuregruppe.

Die kovalente Bindung der Creatinamidinohydrolase an das Polysaccharid zur Herstellung des erfindungsgemäß eingesetzten Stabilisators (Polysaccharid-Enzym-Konjugat) kann nach den bekannten Methoden zur Trägerfixierung von Enzymen erfolgen. Als besonders geeignet hat sich bei Verwendung von Dextran als Polysaccharid die Bindung über Bromcyan oder Trichlortriazin (TCT) erwiesen, wobei zweckmäßig diese Kupplungsmittel zuerst mit dem Dextran umgesetzt werden unter Bildung eines entsprechenden aktivierten Dextrans, welches dann in wäßriger Lösung die Creatinamidinohydrolase kovalent bindet. Polysaccharid und Enzym werden zweckmäßig im Gewichtsverhältnis 1 :1 bis 20, vorzugsweise von 1 :4 bis 15, eingesetzt.

Aufgrund seiner überlegenen Stabilität kann das erfindungsgemäße Sarcosinoxidasepräparat auch bereits mit einem System zur Bestimmung des von der Sarcosinoxidase gebildeten $H_2O_2$ zusammen vorgemischt werden, ebenso wie Puffer und, falls das Präparat im Rahmen der Creatininbestimmung eingesetzt werden soll, auch Creatinamidohydrolase zugesetzt sein können, ohne daß hierdurch die Stabilität herabgesetzt wird.

Als Puffer können im Rahmen der Erfindung alle Puffersubstanzen verwendet werden, die im Rahmen des bekannten Stabilitätsbereiches der Sarc-OD und der Creatinamidinohydrolase puffern. Bevorzugte Beispiele sind Phosphatpuffer und Trispuffer. Der bevorzugte pH-Bereich liegt zwischen 7,0 und 8,5, besonders bevorzugt zwischen 7,6 und 8,2. Geeignete Konzentrationen liegen beispielsweise zwischen 50 und 500 mmol/l.

Als System zur Bestimmung von $H_2O_2$ können die hierfür dem Fachmann bekannten Systeme, die keine die Aktivität von Enzymen beeinträchtigenden Komponenten enthalten, verwendet werden. Typische Beispiele für derartige Systeme können aus H.U. Bergmeyer, Methoden der enzymatischen Analyse, 4. Auflage, entnommen werden. Das Aufhellungssystem kann beispielsweise aus einem nichtionogenen oberflächenaktiven Mittel wie n-Decanolpolyglycolether (Lutensol ON 50®), gegebenenfalls zusammen mit einem anionischen oberflächenaktiven Mittel der Cholsäuregruppe wie Natriumcholat und einem halogenierten Phenol wie 2,4,6-Tribrom-3-hydroxybenzoesäure, bestehen. Schließlich können dem Präparat auch noch übliche Konservierungsmittel wie z. B. Alkaliazide, beigemischt sein.

Gemäß einer weiteren bevorzugten Ausführungsform der Erfindung liegt die Sarcosinoxidase im erfindungsgemäßen Präparat in vorvernetztem Zustand vor. Eine derartige vorvernetzte Sarcosinoxidase läßt sich durch Umsetzung mit bifunktionellen Proteinreagenzien, wie sie beispielsweise in den DE-OS (21 28 743 und 22 60 185 beschrieben sind, erhalten. Besonders bevorzugt wird eine mit Glutardialdehyd vorvernetzte Sarcosinoxidase. Hierdurch läßt sich eine weitere Verbesserung der Stabilität des erfindungsgemäßen Sarcosinoxidasepräparates erreichen.

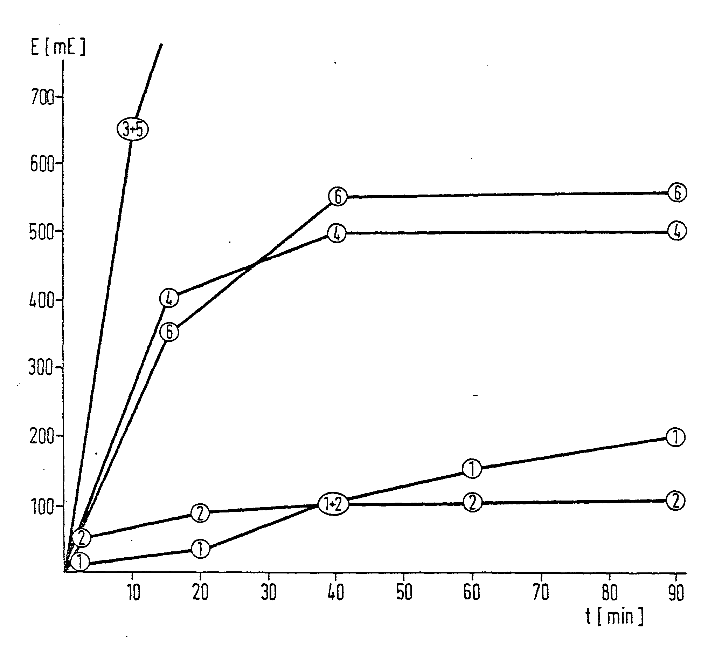

Der durch die Erfindung erzielte Stabilisierungseffekt in Abwesenheit und Gegenwart von oberflächenaktiven Mitteln läßt sich aus der beigefügten Zeichnung ersehen. In dieser ist die als Extinktion bei 546 nm gemessene Trübungsbildung gegen die Inkubationszeit der gelösten Sarcosinoxidase in Minuten bei 37 °C aufgetragen. In allen Fällen wird native Sarcosinoxidase in 300 mM Phosphatpuffer, pH 7,9, eingesetzt. Kurve 1 gilt für Sarc-OD, Kurve 2 für Sarc-OD + Dextran-Enzymkonjugat, Kurve 3 entspricht Kurve 1 mit Zusatz von 0,1 % Lutensol (oberflächenaktives Mittel), Kurve 4 entspricht Kurve 3 mit Zusatz von Dextran-Enzymkonjugat. Kurve 5 entspricht Kurve 3 mit Zusatz von 0,4 % Lutensol® und Kurve 6 entspricht Kurve 5 mit Zusatz von Dextran-Enzymkonjugat. Die Menge an Dextran-Enzymkonjugat betrug dabei in jedem Falle 12 U/ml, die Sarcosinoxidasemenge betrug 6,5 U/ml, die Creatinamidinohydrolase-Konzentration 12 U/ml. Das Verhältnis Enzym : Dextran im Konjugat betrug 1 :4.

Wie die Kurven erkennen lassen, ergibt sich innerhalb von 90 Minuten bei der angegebenen Temperatur für die native Sarcosinoxidase eine Trübung, die bereits etwa doppelt so hoch ist als in Gegenwart des Dextran-Enzym-Konjugats als Stabilisator. In Gegenwart von oberflächenaktiven Mitteln ist die Trübung ohne Stabilisator so stark, daß nach 15 Minuten schon die Meßgrenze überschritten wird. In Gegenwart von oberflächenaktivem Mittel und Stabilisator hingegen tritt zwar anfänglich ebenfalls eine Trübung auf, die jedoch im Meßbereich bleibt und nach etwa 40 Minuten nicht weiter zunimmt. Damit ermöglicht es die Erfindung, ausreichend lange haltbare Reagenzien für die Bestimmung von Sarcosin, Creatin oder Creatinin zu schaffen und damit die Sarcosinbestimmung mittels Sarcosinoxidase und über diese auch die Bestimmung von Creatin und Creatinin in praktisch brauchbare Form zu bringen.

Die folgenden Beispiele erläutern die Erfindung weiter.

Beispiel 1

Herstellung des Stabilisators

Lösliche Dextrane (Firma Roth, Karlsruhe) mit mittleren Molekulargewichten von 10 000, 20 000, 40 000 und 500 000 werden bei pH 10,6 mit Bromcyan umgesetzt.

Hierzu werden 120 g Dextran in 12 l Wasser gelöst und mit 2 n Natronlauge auf pH 10,6 eingestellt. Unter Rühren werden insgesamt 36 bis 60 g Bromcyan in 12 g Portionen innerhalb von 40 Minuten in den Ansatz gegeben. Der pH-Wert wird durch Zugabe von 2 n Natronlauge hierbei auf pH 10,6 gehalten. Die Aktivierungsreaktion ist beendet, wenn keine Natronlauge mehr verbraucht wird.

Die erhaltene klare Lösung wird mit 2 mol/l Salzsäure auf pH 8,0 eingestellt. Das erhaltene aktivierte Dextran wird 2 Stunden bei 20 °C gegen Wasser dialysiert.

44,4 g handelsübliche Creatinamidinohydrolase in lyophilisierter Form (4,2 U/mg ; 0,54 mg Protein/mg Lyophilisat), entsprechend etwa 24 g Enzymprotein, werden in 250 ml 20 mmol/l Phosphatpuffer, pH 8,0, gelöst und gegen 20 l des selben Puffers dialysiert. Die so erhaltene Enzymlösung wird mit der wie oben beschrieben hergestellten Lösung des aktivierten Dextrans vereinigt und bei Raumtemperatur langsam 16 Stunden gerührt. Das erhaltene Dextran-Enzym-Konjugat wird gegen 0,04 mol/l Natriumcitratpuffer, pH 5,8, 3 Stunden dialysiert. Dann werden 24 g Raffinose zugesetzt, die Lösung klarfiltriert und lyophilisiert. 80 % der eingesetzten Enzymaktivität finden sich im Lyophilisat.

Beispiel 2

Durch Zusammenmischen der Reagenzien wird ein stabilisiertes Sarcosinoxidasepräparat hergestellt, welches sich als Reagenz zur Bestimmung von Creatinin eignet und als wäßrige Lösung folgende Zusammensetzung aufweist :

6,5 U/ml Sarcosinoxidase,

12 U/ml Creatinamidinohydrolase-Dextran-Konjugat,

25 U/ml Creatininamidohydrolase

2 U/ml Peroxidase,

2 U/ml Cholesterinesterase aus Candida cylindracea,

150 mmol/l Kaliumphosphatpuffer, pH 7,9, 0,3 % Lutensol ON 50®,

· 8,6 mmol/l 2,4,6-Tribrom-3-hydroxybenzoesäure,

0,8 mmol/l 4-Aminophenazon

5 mmol/l Natriumcholat,

0,5 mmol/l Titriplex III,

0,2 % Natriumazid,

10 μmol/l $K_4Fe(CN)_6$.

Das obige Reagenz wird lyophilisiert. Bei Rekonstitution mit Wasser erhält man eine Lösung, die mindestens 2 Tage für die optische Creatininbestimmung verwendbar bleibt.

**Patentansprüche** (für die Vertragsstaaten : BE, CH, DE, FR, GB, IT, LI, LU, NL, SE)

1. Stabilisiertes Sarcosinoxidase-Präparat, gekennzeichnet durch einen Gehalt an kovalent an wasserlösliches Polysaccharid gebundene Creatinamidinohydrolase.

2. Sarcosinoxidase-Präparat nach Anspruch 1, dadurch gekennzeichnet, daß das Polysaccharid Dextran ist.

3. Sarcosinoxidase-Präparat nach Anspruch 1 oder 2, dadurch gekennzeichnet, daß es ein oberflächenaktives Mittel enthält.

4. Sarcosinoxidase-Präparat nach einem der vorhergehenden Ansprüche, dadurch gekennzeichnet, daß es eine Creatinamidinohydrolase enthält, die an mit Bromcyan oder Trichlortriazin aktiviertes Dextran gebunden ist.

5. Sarcosinoxidase-Präparat nach einem der vorhergehenden Ansprüche, dadurch gekennzeichnet, daß es Creatininamidohydrolase, Puffer und ein System zur Bestimmung von $H_2O_2$ enthält.

6. Verwendung eines stabilisierten Sarcosinoxidase-Präparates nach Anspruch 5 zur Bestimmung von Creatinin.

**Patentansprüche** (für den Vertragsstaat AT)

1. Stabilisiertes Sarcosinoxidase-Präparat, gekennzeichnet durch einen Gehalt an kovalent an wasserlösliches Polysaccharid gebundene Creatinamidinohydrolase.

2. Sarcosinoxidase-Präparat nach Anspruch 1, dadurch gekennzeichnet, daß das Polysaccharid Dextran ist.

3. Sarcosinoxidase-Präparat nach Anspruch 1 oder 2, dadurch gekennzeichnet, daß es ein oberflächenaktives Mittel enthält.

4. Sarcosinoxidase-Präparat nach einem der vorhergehenden Ansprüche, dadurch gekennzeichnet, daß es eine Creatinamidinohydrolase enthält, die an mit Bromcyan oder Trichlortriazin aktiviertes Dextran gebunden ist.

5. Sarcosinoxidase-Präparat nach einem der vorhergehenden Ansprüche, dadurch gekennzeichnet, daß es Creatininamidohydrolase, Puffer und ein System zur Bestimmung von $H_2O_2$ enthält.

6. Verwendung eines stabilisierten Sarcosinoxidase-Präparates nach Anspruch 5 zur Bestimmung von Creatinin.

7. Verfahren zur Herstellung eines stabilisierten Sarcosinoxidase-Präparates, dadurch gekennzeichnet, daß man Sarcosinoxidase mit kovalent an wasserlösliches Polysaccharid gebundener Creatinamidinohydrolase mischt.

**Claims** (for the Contracting States : BE, CH, DE, FR, GB, IT, LI, LU, NL, SE)

1. Stabilised sarcosine oxidase preparation, characterised by a content of creatineamidinohydrolase covalently bound to water-soluble polysaccharide.

2. Sarcosine oxidase preparation according to claim 1, characterised in that the polysaccharide is dextran.

3. Sarcosine oxidase preparation according to claim 1 or 2, characterised in that it contains a surface-active agent.

4. Sarcosine oxidase preparation according to one of the preceding claims, characterised in that it contains a creatineamidinohydrolase which is bound to dextran activated with cyanogen bromide or trichlorotriazine.

5. Sarcosine oxidase preparation according to one of the preceding claims, characterised in that it contains creatinineamidohydrolase, buffer and a system for the determination of $H_2O_2$.

6. Use of a stabilised sarcosine oxidase preparation according to claim 5 for the determination of creatinine.

**Claims** (for the Contracting State AT)

1. Stabilised sarcosine oxidase preparation, characterised by a content of creatineamidinohydrolase covalently bound to water-soluble polysaccharide.

2. Sarcosine oxidase preparation according to claim 1, characterised in that the polysaccharide is dextran.

3. Sarcosine oxidase preparation according to claim 1 or 2, characterised in that it contains a surface-active agent.

4. Sarcosine oxidase preparation according to one of the preceding claims, characterised in that it contains a creatineamidinohydrolase which is bound to dextran activated with cyanogen bromide or trichlorotriazine.

5. Sarcosine oxidase preparation according to one of the preceding claims, characterised in that it contains creatinineamidohydrolase, buffer and a system for the determination of $H_2O_2$.

6. Use of a stabilised sarcosine oxidase preparation according to claim 5 for the determination of creatinine.

7. Process for the preparation of a stabilised sarcosine oxidase preparation, characterised in that one mixes sarcosine oxidase with creatineamidinohydrolase covalently bound to water-soluble polysaccharide.

**Revendications** (pour les Etats contractants : BE, CH, DE, FR, GB, IT, LI, LU, NL, SE)

1. Préparation stabilisée de sarcosine oxydase, caractérisée par une teneur en créatine amidinohydrolase liée de façon covalente à un polysaccharide hydrosoluble.

2. Préparation de sarcosine oxydase selon la revendication 1, caractérisée en ce que le polysaccharide est du dextrane.

3. Préparation de sarcosine oxydase selon la revendication 1 ou 2, caractérisée en ce qu'elle contient un agent tensio-actif.

4. Préparation de sarcosine oxydase selon l'une des revendications précédentes, caractérisée en ce qu'elle contient une créatine amidinohydrolase qui est liée à du dextrane activé par le bromure de cyanogène ou la trichlorotriazine.

5. Préparation de sarcosine oxydase selon l'une des revendications précédentes, caractérisée en ce qu'elle contient de la créatinine amidohydrolase, du tampon et un système pour la détermination de $H_2O_2$.

6. Utilisation d'une préparation de sarcosine oxydase stabilisée selon la revendication 5 pour la détermination de la créatinine.

**Revendications** (pour l'Etat contractant AT)

1. Préparation stabilisée de sarcosine oxydase, caractérisée par une teneur en créatine amidinohydrolase liée de façon covalente à un polysaccharide hydrosoluble.

2. Préparation de sarcosine oxydase selon la revendication 1, caractérisée en ce que le polysaccharide est du dextrane.

3. Préparation de sarcosine oxydase selon la revendication 1 ou 2, caractérisée en ce qu'elle contient un agent tensio-actif.

4. Préparation de sarcosine oxydase selon l'une des revendications précédentes, caractérisée en ce qu'elle contient une créatine amidinohydrolase qui est liée à du dextrane activé au moyen de bromure de cyanogène ou de trichlorotriazine.

5. Préparation de sarcosine oxydase selon l'une des revendications précédentes, caractérisée en ce qu'elle contient de la créatinine amidohydrolase, du tampon et un système pour la détermination de $H_2O_2$.

6. Utilisation d'une préparation de sarcosine oxydase stabilisée selon la revendication 5 pour la détermination de la créatinine.

7. Procédé pour la fabrication d'une préparation stabilisée de sarcosine oxydase, caractérisé en ce que l'on mélange de la sarcosine oxydase avec de la créatine amidinohydrolase liée de façon covalente à un polysaccharide hydrosoluble.